# EUROPEAN PATENT SPECIFICATION

(11) **EP 4 391 840 B1**
(45) Date of publication and mention of the grant of the patent: **06.05.2026**
(21) Application number: 22761017.7
(22) Date of filing: 05.08.2022
(51) Int. Cl.: A24B 15/167, A24F 40/10

(54) **MEDICAL LIQUID COMPOSITION FOR AERIAL ADMINISTRATION**
MEDIZINISCHE FLÜSSIGE ZUSAMMENSETZUNG ZUR VERABREICHUNG IN DER LUFT
COMPOSITION LIQUIDE MÉDICALE POUR ADMINISTRATION AÉRIENNE

(30) Priority: 23.08.2021 IT 202100022172
(43) Date of publication of application: 03.07.2024
(73) Proprietor: Natural Academy S.r.l., 22066 Mariano Comense (CO) (IT); Ape8 S.r.l., 21040 Origgio (VA) (IT)
(72) Inventor: FERRI, Emanuele, 23876 Monticello Brianza, Lecco (IT); DELL'AGLI, Mario, 20133 Milano (IT); FERRI, Nicola, 35136 Padova (IT)
(74) Representative: Long, Giorgio
(86) International application number: PCT/IB2022/057304
(87) International publication number: WO 2023/026121

(56) References cited:
- WO-A1-2019/046664
- WO-A1-2021/127101
- WO-A2-2015/148649
- CN-B- 103 370 307
- KR-A- 20210 075 107

## Description

### Field of application of the invention

The present invention relates to a medical liquid composition with protective and antioxidant properties, which is usable for aerial administration, for example with personal vaporizers and aerosol devices and to devices for aerial administration containing such composition.

### Background art

The damage to health caused by tobacco smoke is universally known. It is well known that cigarette smoking causes serious damage to health, not so much due to nicotine, which is also a highly toxic substance, but due to the combustion by-products, such as polycyclic aromatic hydrocarbons, tar and carbon monoxide, some of which have a pronounced carcinogenic effect.

Alternative smoking is now a widespread practice all over the world, at least partially replacing cigarette smoking. There are two types of alternative smoking on the market. A first type is the so-called heat-not-burn systems which include heating tobacco at a controlled temperature (generally no higher than 350°C), so as to avoid the harmful effects of high-temperature combustion. The second type is so-called "electronic cigarettes" which vaporize a liquid comprising, in addition to a base adapted to provide a sufficiently dense body to the vapor released, also a controlled amount of nicotine and various types of flavorings.

The first type, with heated tobacco, is more similar to traditional smoking, but some toxic substances contained in tobacco in addition to nicotine are still released despite the lower combustion temperatures. A typical example is nitrosamines.

Electronic cigarettes are safer, since they do not contain such toxic or carcinogenic substances, apart from a controlled amount of nicotine, which can be added to the composition to provide the smoker with the desired nicotinic receptor stimulating effect. The possibility of flavoring the composition in various manners is a further attraction.

However, electronic cigarettes too are not free of toxicity. In fact, the basic composition mostly consists of a mixture of propylene glycol and glycerol which cause, especially the former, damage to the cells of the oropharyngeal mucosa and respiratory tract.

Smokers of both traditional cigarettes and alternative products generally have two different smoking modes: the so-called "cheek draw" and the "lung draw". In the first case the smoke is not ingested but remains in the oral cavity, while in the second case it is inhaled up to the respiratory tract. The damage of the smoke can thus be visible at the level of both the latter and the oropharyngeal cavity.

Therefore, it is the problem addressed by the present invention to provide a composition for aerial administration which can counteract and alleviate the damage caused by the chronic use of cigarettes, heated tobacco and electronic cigarettes at the level of both the oral cavity and pulmonary respiratory tract. Document WO2015/148649 discloses a composition comprising a nicotine-containing material and an anti-cancer agent dissolved in a solvent suitable to be administered with an inhalation device.

### Summary of the invention

The aforesaid technical problem is substantially solved by a composition comprising the technical features set out in one or more of the appended claims, the definitions of which form an integral part of the present description for the purpose of sufficiency of description.

Therefore, the present invention relates to a composition for aerial administration comprising or consisting of the following components:

| | |
|---|---|
| a) water | 20-50% by weight |
| b) a diol | 20-60% by weight |
| c) glycerol | 10-30% by weight |
| d) ethanol | 0-10% by weight |
| e) one or more protective substances preferably selected from cyclodextrins and cellulose derivatives; | |
| f) one or more antioxidants; | |
| g) optionally, a preservative, | |

in which the sum of the weight percentages of components e), f) and g) is between 0.1% and 5% by weight.

Further features and advantages of the present invention will become more apparent from the indicative and thus non-limiting description of preferred, but not exclusive embodiments of the invention.

### Brief description of the drawings

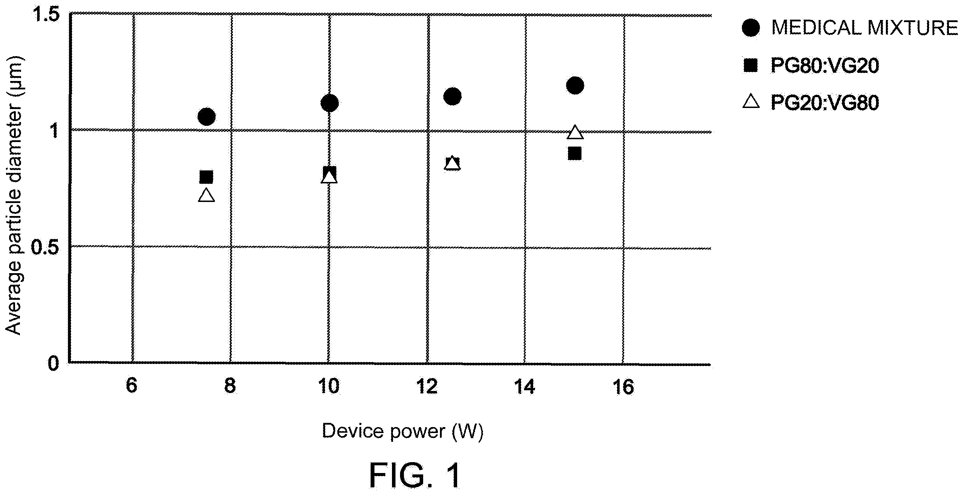

Figure 1 depicts a diagram comparing a composition according to the invention (MEDICAL MIXTURE) and two compositions of the prior art (80PG/20VG and 20PG/80VG), depicting the variation of the average size of the vapor droplets as a function of the electrical power applied by dispensing the compositions with a common electronic cigarette.

### Detailed description of the invention

The present invention relates to a composition for aerial administration comprising or consisting of the following components:

| | |
|---|---|
| a) water | 10-50% or 20-50% by weight |
| b) a diol | 20-65% or 20-60% by weight |
| c) glycerol | 10-40% or 10-30% by weight |
| d) ethanol | 0-10% by weight |
| e) one or more protective substances selected from linear or branched dextrins, cyclodextrins and cellulose | |
| derivatives; | |
| f) one or more antioxidants; | |
| g) optionally, a preservative, | |

in which the sum of the weight percentages of components e), f) and g) is between 0.1% and 5% by weight.

The diol can be a C2-C4 alkyldiol or a C2-C4-dialkylether diol.

The invention further relates to a composition for aerial administration, which preferably does not contain propylene glycol, comprising or consisting of the following components:

| | |
|---|---|
| a) water | 10-50% or 20-50% by weight |
| b) 1,3-propanediol | 20-65% or 20-60% by weight |
| c) glycerol | 10-40% or 10-30% by weight |
| d) ethanol | 0-10% by weight |
| e) one or more protective substances selected from linear or branched dextrins, cyclodextrins and cellulose derivatives; | |
| f) one or more antioxidants; | |
| g) optionally, a preservative, | |

in which the sum of the weight percentages of components e), f) and g) is between 0.1% and 5% by weight.

The water is preferably purified water FU. 1,3-propanediol (component (b)) serves as a viscosifying agent and carrier of bioactive substances.

The one or more protective substances (component e)) are more preferably selected from cyclodextrins, carboxymethyl cellulose and microcrystalline cellulose.

The one or more antioxidants (component f)) are preferably selected from hydroxytyrosol, curcumin, gallic acid, stilbenes including resveratrol and piceid, ellagic acid, caffeic acid derivatives including caffeic acid, chlorogenic and rosmarinic acid, secoiridoids such as oleuropein and ligstroside and glycoside derivatives, flavonoid derivatives including epigallocatechin gallate, quercetin, luteolin, apigenin, catechin and glycoside derivatives thereof. The preservative (component g)) is preferably selected from sodium benzoate and sodium ascorbate.

In preferred embodiments, the composition of the invention does not contain propylene glycol and comprises or consists of the following components:

| | |
|---|---|
| a) water | 10-40%, or 30-40%, or 32-38% by weight |
| b) 1,3-propanediol | 30-65%, or 30-50%, or 40-48% by weight |
| c) glycerol | 15-40%, or 15-25% by weight |
| d) ethanol | 0-3% by weight |
| e) one or more protective substances selected from linear or branched dextrins and cyclodextrins; | |
| f) one or more antioxidants selected from ellagic acid, rosmarinic acid and resveratrol; | |
| g) optionally, a preservative, | |

in which the sum of the weight percentages of components e), f) and g) is between 0.2% and 0.7%, or between 0.3 and 0.6% by weight.

The term "composition for aerial administration" means a composition which can be dispensed either by conventional aerosol systems or by portable vaporizers or common electronic cigarettes. This latter dispensing method is in certain cases the preferred one, as it can be more familiar to smokers, who are the main users of the composition of the invention.

The composition for aerial administration according to the invention, by virtue of the weight ratios of the main components, i.e., water, 1,3-propanediol and glycerol which form a basic composition, is in fact adapted to be dispensed through a portable vaporization system, since it provides an aerosol with appropriate structure and density if vaporized through both a personal vaporizer and a common electronic cigarette.

A parameter which is of some importance is the average size of the vapor droplets dispensed, since, depending on the average size of the aerosol droplets, the composition of the invention can stop in the oral cavity, or can be inhaled up to the pulmonary level.

Particles of size exceeding 1 micron are known to stop mainly in the oropharyngeal cavity, while particles of size less than 1 micron can penetrate up to the pulmonary respiratory tract.

Figure 1 shows a diagram comparing a composition according to the invention (indicated as MEDICAL MIXTURE and comprising 1,3-propanediol 60% by weight, purified water 29.6% by weight, glycerol 10% by weight, other components as defined above in points e)-g) 0.4% by weight) and two conventional vaping compositions containing respectively 80% by weight propylene glycol/20% by weight glycerol (80PG/20VG) and 20% by weight propylene glycol/80% by weight glycerol (20PG/80VG).

In all cases, using a new-generation vaping device provided with a power control circuit, an atomizer with air regulated to the maximum opening and a resistance of 1.5 Ohm and operating with an electric power varying from 7.5 to 15 Watts, an increase in the size of the vapor droplets dispensed (the average diameter of the particles in microns is shown in the ordinate) as the electric power increases is noted. However, while the compositions of the prior art involve dispensing droplets of average diameter from 0.7 to about 0.9 microns, with the composition of the invention the average particle size is from 1.1 to 1.25 microns.

This means that, while the vaping compositions of the prior art easily reach the pulmonary respiratory tract, the composition according to the invention which has been tested will stop at the oropharyngeal level, exerting the protective filming and antioxidant action at this level. With the composition of the invention, it will be possible to obtain a droplet size of less than 1 micron, suitable for the medical treatment of the pulmonary respiratory tract, acting on the following parameters:
i) increase in the proportion of water up to the limit of 50% by weight, and/or
ii) addition of ethanol within the limit of 10% by weight, and/or
iii) decrease in the weight percentage of 1,3-propanediol and/or increase in the weight percentage of glycerol within the limits defined above, and/or
iv) increase in the airflow of the dispensing device, and/or
v) use of atomizer nozzles with a hole of smaller diameter.

For example, a 6:1 1,3-propanediol/glycerol weight ratio will produce larger droplets of size exceeding than 1 micron, while a 2:3 weight ratio will produce droplets of a size close to 1 micron or less.

A composition suitable for treating a smoker's oropharyngeal cavity comprises, for example:

| | |
|---|---|
| water | <40% by weight |
| 1,3-propanediol | >40% by weight |
| glycerol | ≤20% by weight. |

Outside these ranges, the composition will therefore be suitable for a treatment of the pulmonary respiratory tract.

The increase in air flow (point iv) of the dispensing device is normally an adjustable parameter on the device used.

The size of the nozzle hole (point v) is a typical feature of the device, depending on whether it is dedicated to a cheek or lung draw.

Flavors can be added to the composition as outlined above, in a percentage by volume less than or equal to 10% by weight.

The flavors can be fruit, tobacco, mentholated flavors or mixtures thereof, as long as the safety thereof has been proven and as long as they do not interfere with the purpose of the medical mixture to exert a filming, protective and antioxidant action on oral mucosa and respiratory tract.

Therefore, the composition of the invention can be used for both a treatment of the oropharyngeal cavity and a treatment of the pulmonary respiratory tract by changing the composition within the claimed intervals and possibly operating on parameters of the dispensing device used. The choice of one or other type of composition and device will depend on the needs of the smoker, i.e., depending on whether he/she is used to a cheek or lung draw.

### EXPERIMENTAL SECTION

We conducted experiments to evaluate and compare, at a preliminary level in vitro, the adhesive capacity of three topical formulations (oral sprays) by verifying the effectiveness of nebulization, the formation of droplets, the size and permanence thereof on a rough surface which mimics skin. The support chosen to perform the tests is polymethylmethacrylate, PMMA plates with specific surface roughness.

3 samples having the following formulations (weight percentages) were tested:

| | **Ex**. **1** | **Ex**. **2** | **Comparison** |
|---|---|---|---|
| Water | 28.5% | 28.5% | 28.75% |
| Propylene glycol | - | - | 25% |
| 1,3-Propanediol | 45% | 45% | - |
| Glycerol | 25% | 20% | 5% |
| Ethanol | - | 5% | 40% |
| Carboxymethyl cellulose | - | - | 0.25% |
| Cyclodextrin | 0.5% | 0.5% | - |
| Antioxidant | 1% | 1% | 1% |
| | 100% | 100% | 100% |

The support used for the evaluations is PMMA WW5 Schonberg GmbH plates (Munich, Germany) with an area of 25 cm2, characterized by an average surface roughness of 4.8 ± 1.4 µm.

The cyclodextrin used was alpha cyclodextrin (CAS # 10016-20-3) .

The antioxidant used was resveratrol (CAS# 501-36-0).

Each sample was nebulized 2 times at a distance of 20 cm on PMMA plates positioned at an inclination of 45° and observed in such a position for 30 minutes following the nebulization; after 30 minutes the plates were tilted to 60° and observed for another 20 minutes.

The formulation of example 1 showed good nebulization efficiency with formation of very small droplets on the PMMA plates, homogeneously distributed on the surface. There was no slipping in the first 30 minutes of observation of the plates positioned at 45°. A barely perceptible slipping was evidenced after 20 min of observation of the plates positioned at 60°.

The formulation of Example 2 showed good nebulization efficiency with formation of small droplets on the PMMA plates, homogeneously distributed on the surface. There was no slipping in the first 30 minutes of observation of the plates positioned at 45°. In the next 20 min of observation of the plates positioned at 60°, a slight slipping was observed with coalescence of some droplets to form larger droplets.

The comparison formulation (prior art) showed a "jet" dispensing with immediate slipping of the formulation on the PMMA plates. Therefore, it was not possible to observe the behavior for 30 min on the PMMA plates positioned at 45° and for a further 20 min on the PMMA plates positioned at 60°.

The experiments have revealed that the formulations according to the invention allow a much better nebulization than the comparison, known from the prior art. In particular, the formulation of example 1 showed absolutely better adhesive properties on PMMA plates (inclination at 45° and 60°). In fact, the formulation of example 1 shows a greater nebulization effectiveness with formation of very small droplets which are homogeneously distributed on the surface of the PMMA plates, to which they remain effectively adhered.

The composition of the invention can be administered 1 to 10 times a day, ideally before being exposed to pollutants, toxic and oxidizing substances, with the caution not to exceed the indications provided.

The composition of the invention thus allows protecting and alleviating the damage caused to a smoker by conventional cigarette smoking, vapors produced by heated tobacco, or vapors of electronic cigarettes.

The composition of the invention is also suitable for a tobacco product cessation treatment, comprising nicotine. The invention further relates to a device for aerial administration containing one or more doses of one or more compositions according to the invention, where the device is selected from a personal vaporizer, an electronic cigarette and an aerosol atomizer.

## Claims

1. A composition for aerial administration comprising or consisting of the following components:
| | |
|---|---|
| a) water | 10-50% or 20-50% by weight |
| b) a diol | 20-65% or 20-60% by weight |
| c) glycerol | 10-40% or 10-30% by weight |
| d) ethanol | 0-10% by weight |
| e) one or more protective substances selected from linear or branched dextrins, cyclodextrins and cellulose derivatives; | |
| f) one or more antioxidants; | |
| g) optionally, a preservative, | |
wherein the sum of the weight percentages of components e), f) and g) is between 0.1% and 5% by weight.

2. The composition for aerial administration according to claim 1, comprising or consisting of the following components:
| | |
|---|---|
| a) water | 10-50% or 20-50% by weight |
| b) 1,3-propanediol | 20-65% or 20-60% by weight |
| c) glycerol | 10-40% or 10-30% by weight |
| d) ethanol | 0-10% by weight |
| e) one or more protective substances selected from linear or branched dextrins, cyclodextrins and cellulose derivatives; | |
| f) one or more antioxidants; | |
| g) optionally, a preservative, | |
wherein the sum of the weight percentages of components e), f) and g) is between 0.1% and 5% by weight.

3. The composition for aerial administration according to claim 2, which does not contain propylene glycol and comprises or consists of the following components:
| | |
|---|---|
| a) water | 10-50% or 20-50% by weight |
| b) 1,3-propanediol | 20-65% or 20-60% by weight |
| c) glycerol | 10-40% or 10-30% by weight |
| d) ethanol | 0-10% by weight |
| e) one or more protective substances selected from linear or branched dextrins, cyclodextrins and cellulose derivatives; | |
| f) one or more antioxidants; | |
| g) optionally, a preservative, | |
wherein the sum of the weight percentages of components e), f) and g) is between 0.1% and 5% by weight.

4. The composition according to claim 2 or 3, comprising or consisting of the following components:
| | |
|---|---|
| a) water | 10-40%, or 30-40%, or 32-38% by weight |
| b) 1,3-propanediol | 30-65%, or 30-50%, or 40-48% by weight |
| c) glycerol | 15-40%, or 15-25% by weight |
| d) ethanol | 0-10% by weight |
| e) one or more protective substances selected from linear or branched dextrins and cyclodextrins; | |
| f) one or more antioxidants; | |
| g) optionally, a preservative, | |
wherein the sum of the weight percentages of components e), f) and g) is between 0.2% and 0.7%, or between 0.3 and 0.6% by weight.

5. The composition according to any one of claims 1 to 4, wherein the one or more protective substances of component e) are selected from cyclodextrins, carboxymethyl cellulose and microcrystalline cellulose.

6. The composition according to any one of claims 1 to 5, wherein the one or more antioxidants of component f) are selected from hydroxytyrosol, curcumin, gallic acid, stilbenes including resveratrol and piceid, ellagic acid, caffeic acid derivatives including caffeic acid, chlorogenic and rosmarinic acid, secoiridoids such as oleuropein and ligstroside and glycoside derivatives, flavonoid derivatives including epigallocatechin gallate, quercetin, luteolin, apigenin, catechin and glycoside derivatives thereof.

7. The composition according to any one of claims 1 to 6, wherein the preservative is sodium benzoate or sodium ascorbate.

8. The composition according to any one of claims 1 to 7, comprising flavors preferably selected from fruit, tobacco, mentholated flavors, or mixtures thereof.

9. The composition according to any one of claims 1 to 8, comprising:
| | |
|---|---|
| water | <40% by weight |
| 1,3-propanediol | >40% by weight |
| glycerol | ≤40% by weight. |

10. The composition according to any one of claims 1 to 9, comprising nicotine.

11. A device for aerial administration containing one or more doses of one or more compositions according to any one of claims 1 to 10, wherein said device is selected from a personal vaporizer, an electronic cigarette and an aerosol atomizer.

## Patentansprüche

1. Zusammensetzung zur Verabreichung über die Luft, die die folgenden Bestandteile umfasst oder daraus besteht:
| | |
|---|---|
| a) Wasser | 10-50 Gew.-% oder 20-50 Gew.-% |
| b) ein Diol | 20-65 Gew.-% oder 20-60 Gew.-% |
| c) Glycerin | 10-40 Gew.-% oder 10-30 Gew.-% |
| d) Ethanol | 0-10 Gew.-% |
| e) eine oder mehrere Schutzsubstanzen, ausgewählt aus linearen oder verzweigten Dextrinen, Cyclodextrinen und Cellulosederivaten; | |
| f) ein oder mehrere Antioxidantien; | |
| g) gegebenenfalls ein Konservierungsmittel, | |
wobei die Summe der Gewichtsprozente der Bestandteile e), f) und g) zwischen 0,1 Gew.-% und 5 Gew.-% liegt.

2. Zusammensetzung zur Verabreichung über die Luft nach Anspruch 1, die die folgenden Bestandteile umfasst oder daraus besteht:
| | |
|---|---|
| a) Wasser | 10-50 Gew.-% oder 20-50 Gew.-% |
| b) 1,3-Propandiol | 20-65 Gew.-% oder 20-60 Gew.-% |
| c) Glycerin | 10-40 Gew.-% oder 10-30 Gew.-% |
| d) Ethanol | 0-10 Gew.-% |
| e) eine oder mehrere Schutzsubstanzen, ausgewählt aus linearen oder verzweigten Dextrinen, Cyclodextrinen und Cellulosederivaten; | |
| f) ein oder mehrere Antioxidantien; | |
| g) gegebenenfalls ein Konservierungsmittel, wobei die Summe der Gewichtsprozente der Bestandteile e), f) und g) zwischen 0,1 Gew.-% und 5 Gew.-% liegt. | |

3. Zusammensetzung zur Verabreichung über die Luft nach Anspruch 2, die kein Propylenglykol enthält und die folgenden Bestandteile umfasst oder daraus besteht:
| | |
|---|---|
| a) Wasser | 10-50 Gew.-% oder 20-50 Gew.-% |
| b) 1,3-Propandiol | 20-65 Gew.-% oder 20-60 Gew.-% |
| c) Glycerin | 10-40 Gew.-% oder 10-30 Gew.-% |
| d) Ethanol | 0-10 Gew.-% |
| e) eine oder mehrere Schutzsubstanzen, ausgewählt aus linearen oder verzweigten Dextrinen, Cyclodextrinen und Cellulosederivaten; | |
| f) ein oder mehrere Antioxidantien; | |
| g) gegebenenfalls ein Konservierungsmittel, wobei die Summe der Gewichtsprozente der Bestandteile e), f) und g) zwischen 0,1 Gew.-% und 5 Gew.-% liegt. | |

4. Zusammensetzung nach Anspruch 2 oder 3, die die folgenden Bestandteile umfasst oder daraus besteht:
| | |
|---|---|
| a) Wasser | 10-40 Gew.-% oder 30-40 Gew.-% oder 32-38 Gew.-% |
| b) 1,3-Propandiol | 30-65 Gew.-% oder 30-50 Gew.-% oder 40-48 Gew.-% |
| c) Glycerin | 15-40 Gew.-% oder 15-25 Gew.-% |
| d) Ethanol | 0-10 Gew.-% |
| e) eine oder mehrere Schutzsubstanzen, ausgewählt aus linearen oder verzweigten Dextrinen und Cyclodextrinen; | |
| f) ein oder mehrere Antioxidantien; | |
| g) gegebenenfalls ein Konservierungsmittel, wobei die Summe der Gewichtsprozente der Bestandteile e), f) und g) zwischen 0,2 Gew.-% und 0,7 Gew.-% oder zwischen 0,3 Gew.-% und 0,6 Gew.-% liegt. | |

5. Zusammensetzung nach einem der Ansprüche 1 bis 4, wobei die eine oder mehreren Schutzsubstanzen des Bestandteils e) aus Cyclodextrinen, Carboxymethylcellulose und mikrokristalliner Cellulose ausgewählt sind.

6. Zusammensetzung nach einem der Ansprüche 1 bis 5, wobei das eine oder die mehreren Antioxidantien des Bestandteils f) aus Hydroxytyrosol, Curcumin, Gallussäure, Stilbenen, einschließlich Resveratrol und Piceid, Ellagsäure, Kaffeesäurederivaten, einschließlich Kaffeesäure, Chlorogensäure und Rosmarinsäure, Secoiridoiden wie Oleuropein und Ligstrosid und Glycosidderivaten, Flavonoidderivaten, einschließlich Epigallocatechingallat, Quercetin, Luteolin, Apigenin, Catechin und Glycosidderivaten davon ausgewählt sind.

7. Zusammensetzung nach einem der Ansprüche 1 bis 6, wobei das Konservierungsmittel Natriumbenzoat oder Natriumascorbat ist.

8. Zusammensetzung nach einem der Ansprüche 1 bis 7, umfassend Geschmacksstoffe, vorzugsweise ausgewählt aus Frucht, Tabak, mentholhaltigen Geschmacksstoffen oder Mischungen davon.

9. Zusammensetzung nach einem der Ansprüche 1 bis 8, umfassend:
| | |
|---|---|
| Wasser | < 40 Gew.-% |
| 1,3-Propandiol | > 40 Gew.-% |
| Glycerin | ≤ 40 Gew.-%. |

10. Zusammensetzung nach einem der Ansprüche 1 bis 9, die Nikotin umfasst.

11. Vorrichtung zur Verabreichung über die Luft, die eine oder mehrere Dosen einer oder mehrerer Zusammensetzungen nach einem der Ansprüche 1 bis 10 enthält, wobei die Vorrichtung aus einem persönlichen Verdampfer, einer elektronischen Zigarette und einem Aerosolzerstäuber ausgewählt ist.

## Revendications

1. Composition pour administration aérienne comprenant ou constituée des composants suivants :
| | |
|---|---|
| a) eau | 10-50 % ou 20-50 % en poids |
| b) un diol | 20-65 % ou 20-60 % en poids |
| c) glycérol | 10-40 % ou 10-30 % en poids |
| d) éthanol | 0-10 % en poids |
| e) une ou plusieurs substances protectrices sélectionnées parmi les dextrines linéaires ou ramifiées, les cyclodextrines et les dérivés de la cellulose ; | |
| f) un ou plusieurs antioxydants ; | |
| g) éventuellement, un conservateur, | |
dans laquelle la somme des pourcentages pondéraux des composants e), f) et g) est comprise entre 0,1 % et 5 % en poids.

2. Composition pour administration aérienne selon la revendication 1, comprenant ou constituée des composants suivants :
| | |
|---|---|
| a) eau | 10-50 % ou 20-50 % en poids |
| b) 1,3-propanediol | 20-65 % ou 20-60 % en poids |
| c) glycérol | 10-40 % ou 10-30 % en poids |
| d) éthanol | 0-10 % en poids |
| e) une ou plusieurs substances protectrices sélectionnées parmi les dextrines linéaires ou ramifiées, les cyclodextrines et les dérivés de la cellulose ; | |
| f) un ou plusieurs antioxydants ; | |
| g) éventuellement, un conservateur, dans la quelle la somme des pourcentages en poids des composants e), f) et g) est comprise entre 0,1 % et 5 % en poids. | |

3. Composition pour administration aérienne selon la revendication 2, qui ne contient pas de propylène glycol et comprend ou est constituée des composants suivants :
| | |
|---|---|
| a) eau | 10-50 % ou 20-50 % en poids |
| b) 1,3-propanediol | 20-65 % ou 20-60 % en poids |
| c) glycérol | 10-40 % ou 10-30 % en poids |
| d) éthanol | 0-10 % en poids |
| e) une ou plusieurs substances protectrices sélectionnées parmi les dextrines linéaires ou ramifiées, les cyclodextrines et les dérivés de la cellulose ; | |
| f) un ou plusieurs antioxydants ; | |
| g) éventuellement, un conservateur, dans la quelle la somme des pourcentages en poids des composants e), f) et g) est comprise entre 0,1 % et 5 % en poids. | |

4. Composition selon la revendication 2 ou 3, comprenant ou constituée des composants suivants :
| | |
|---|---|
| a) eau | 10-40 %, ou 30-40 %, ou 32-38 % en poids |
| b) 1,3-propanediol | 30-65 %, ou 30-50 %, ou 40-48 % en poids |
| c) glycérol | 15-40 %, ou 15-25 % en poids |
| d) éthanol | 0-10 % en poids |
| e) une ou plusieurs substances protectrices sélectionnées parmi | |
| les dextrines linéaires ou ramifiées et les cyclodextrines ; | |
| f) un ou plusieurs antioxydants ; | |
| g) éventuellement, un conservateur, dans la quelle la somme des pourcentages en poids des composants e), f) et g) est comprise entre 0,2 % et 0,7 %, ou entre 0,3 et 0,6 % en poids. | |

5. Composition selon l'une quelconque des revendications 1 à 4, dans laquelle la ou les substances protectrices du composant e) sont sélectionnées parmi les cyclodextrines, la carboxyméthylcellulose et la cellulose microcristalline.

6. Composition selon l'une quelconque des revendications 1 à 5, dans laquelle le ou les antioxydants du composant f) sont sélectionnés parmi l'hydroxytyrosol, la curcumine, l'acide gallique, les stilbènes y compris le resvératrol et le picéide, l'acide ellagique, les dérivés de l'acide caféique y compris l'acide caféique, l'acide chlorogénique et l'acide rosmarinique, les sécoiridoïdes tels que l'oleuropéine et les dérivés de ligstroside et de glycoside, dérivés de flavonoïdes y compris le gallate d'épigallocatéchine, la quercétine, la lutéoline, l'apigénine, la catéchine et leurs dérivés glycosidiques.

7. Composition selon l'une quelconque des revendications 1 à 6, dans laquelle le conservateur est du benzoate de sodium ou de l'ascorbate de sodium.

8. Composition selon l'une quelconque des revendications 1 à 7, comprenant des arômes de préférence sélectionnés parmi les fruits, le tabac, les arômes mentholés ou leurs mélanges.

9. Composition selon l'une quelconque des revendications 1 à 8, comprenant :
| | |
|---|---|
| eau | < 40 % en poids |
| 1,3-propanediol | > 40 % en poids |
| glycérol | ≤ 40 % en poids. |

10. Composition selon l'une quelconque des revendications 1 à 9, comprenant de la nicotine.

11. Dispositif d'administration aérienne contenant une ou plusieurs doses d'une ou de plusieurs compositions selon l'une quelconque des revendications 1 à 10, ledit dispositif étant sélectionné parmi un vaporisateur personnel, une cigarette électronique et un atomiseur aérosol.
